# EUROPÄISCHE PATENTSCHRIFT

(11) **EP 2 097 752 B1**
(45) Veröffentlichungstag und Bekanntmachung des Hinweises auf die Patenterteilung: **18.05.2011**
(21) Anmeldenummer: 07819658.1
(22) Anmeldetag: 07.11.2007
(51) Int. Cl.: G01N 33/543, C12N 5/00

(54) **VERFAHREN ZUR AUFREINIGUNG WENIGSTENS EINER NACHZUWEISENDEN ZIELSUBSTANZ**
METHOD FOR THE PURIFICATION OF AT LEAST ONE TARGET SUBSTANCE THAT IS TO BE IDENTIFIED
PROCÉDÉ DE PURIFICATION D'AU MOINS UNE SUBSTANCE CIBLE À METTRE EN ÉVIDENCE

(30) Priorität: 08.11.2006 DE 102006052923; 21.12.2006 DE 102006060717
(43) Veröffentlichungstag der Anmeldung: 09.09.2009
(60) Teilanmeldung: 10173987.8
(73) Patentinhaber: Peter, Jochen, 82362 Weilheim (DE)
(72) Erfinder: Peter, Jochen, 82362 Weilheim (DE)
(74) Vertreter: Sandmann, Wolfgang
(86) Internationale Anmeldenummer: PCT/EP2007/009648
(87) Internationale Veröffentlichungsnummer: WO 2008/055671

(56) Entgegenhaltungen:
- US-A- 6 165 710
- JOCHEN PETER ET AL: "PURIFICATION OF PROSTATE-SPECIFIC ANTIGEN FROM HUMAN SERUM BY INDIRECT IMMUNOSORPTION AND ELUTION WITH A HAPTEN" ANALYTICAL BIOCHEMISTRY, ACADEMIC PRESS, SAN DIEGO, CA, US, Bd. 273, 1999, Seiten 98-104, XP002945712 ISSN: 0003-2697
- PETER J ET AL: "Identification of precursor forms of free prostate-specific antigen in serum of prostate cancer patients by immunosorption and mass spectrometry" CANCER RESEARCH, AMERICAN ASSOCIATION FOR CANCER RESEARCH, BALTIMORE, MD, US, Bd. 61, Nr. 3, 1. Februar 2001 (2001-02-01), Seiten 957-962, XP002341862 ISSN: 0008-5472
- GOTTSCHALK W K ET AL: "PURIFICATION OF A NATURALLY PRODUCED LOW MOLECULAR WEIGHT ORGANIC FACTOR THAT REVERSIBLY BLOCKS ENCYSTMENT OF BLASTOCLADIELLA-EMERSONI ZOOSPORES" JOURNAL OF BIOLOGICAL CHEMISTRY, Bd. 260, Nr. 11, 1985, Seiten 6588-6591, XP002473654 ISSN: 0021-9258
- DESPA FLORIN: "Biological water: Its vital role in macromolecular structure and function." ANNALS OF THE NEW YORK ACADEMY OF SCIENCES DEC 2005, Bd. 1066, Dezember 2005 (2005-12), Seiten 1-11, XP002487698 ISSN: 0077-8923
- LAMBERT K ET AL: "GROWTH OF HUMAN DIPLOID CELLS (STRAIN MRC-5) IN DEFINED MEDIUM; REPLACEMENT OF SERUM BY A FRACTION OF SERUM ULTRAFILTRATE" JOURNAL OF CELL SCIENCE, CAMBRIDGE UNIVERSITY PRESS, LONDON, GB, Bd. 35, 1. Januar 1979 (1979-01-01), Seiten 381-392, XP001015663 ISSN: 0021-9533
- ARCHER ET AL: "Magnetic bead-based solid phase for selective extraction of genomic DNA" ANALYTICAL BIOCHEMISTRY, ACADEMIC PRESS INC. NEW YORK, Bd. 355, Nr. 2, 15. August 2006 (2006-08-15), Seiten 285-297, XP005569547 ISSN: 0003-2697

## Beschreibung

### Technisches Gebiet

Die Erfindung bezieht sich auf ein Verfahren zur Aufreinigung wenigstens einer nachzuweisenden Zielsubstanz, die während einer Zellkultivierung in einem Zellkulturmedium auftritt oder gebildet wird, bei dem zu dem Zellkulturmedium magnetische, an ihrer Oberfläche funktionalisierte Partikel, so genannte Beads, zugegeben werden, an deren Oberfläche sich die Zielsubstanz selektiv anlagert und durch Applikation eines Magnetfeldes die mit der Zielsubstanz behafteten Partikel aus dem Zellkulturmedium selektiert werden.

### Stand der Technik

Gattungsgemäße Verfahren werden bevorzugt zur präparativen Isolierung von Substanzen, welche von Zellen in ein Zellkulturmedium abgegeben werden eingesetzt, um Zelleigenschaften sowie auch ihr weiteres Wachstumsverhalten studieren zu können. Von besonderem Interesse ist dabei die Erforschung von Tumorzellen, deren unkontrolliertes Zellwachstum verantwortlich für die häufigste Todesursache beim Menschen ist.

Für eine verantwortungsvolle Therapie zur Bekämpfung eines beim Menschen festgestellten Tumors ist es unablässig zunächst die Tumorart zu bestimmen, bspw. handelt es sich um eine nicht invasive, invasive Tumorart oder um ductales Carcinoma in situ, etc.. Die Art des Tumors ist hierbei entscheidend für den weiteren Verlauf der Therapie. Hintergrund dabei ist, dass Tumorzellen im Laufe der Progression ihre Eigenschaften verändern. So besitzt bspw. die nicht invasive Krebszelllinie MCF-7 einen Östrogenrezeptor, so dass diese Art von Tumore mit einem Inhibitor des Östrogenrezeptors, z.B. mit Tamixofen, behandelt werden können. Im Wege der Progression kann eine Umwandlung einer gutartigen, d.h. nicht invasiven Tumorzelle in einen bösartigen, d.h. invasiven, Zelltyp erfolgen, bei dem der Östrogenrezeptor an der Zelloberfläche nicht mehr präsentiert wird und daher eine Hormonhandlung nicht mehr möglich ist.

Grundsätzlich wird die Progression eines Tumors durch Mutationen ausgelöst, die wiederum durch äußere Einflüsse wie z.B. Sauerstoffarmut (Hypoxie) hervorgerufen werden können. Neben der Zelleigenschaft, dass Tumorzellen nicht nur Rezeptoren an ihrer Oberfläche vorsehen, die in Art, Wirkung und Anzahl aufgrund der Mutation starken Veränderungen unterliegen, verändern Tumorzellen aber auch ihr Verhalten in der Sekretion von verschiedenen regulatorischen Peptiden und Proteinen.

Ein Ansatz zur Findung von Tumormarkern aus Serum/Plasma heraus wird in der modernen Proteomforschung unter der Verwendung verschiedener chromatographischer Schritte, der Verwendung von 2-D-Gelelektrophorese und anschließender massenspektrometrischer Methoden durchgeführt. Gegenwärtig ist jedoch aufgrund von sehr hoch konzenrtierten Proteinen im Serum/Plasma die Suche nach potentiellen Markern durch Überlagerungseffekte ins Stocken geraten. Aus diesem Grunde werden Untersuchungen mit Tumorzelllinien durchgeführt. Derartige Untersuchungen an Tumorzellen haben aber gezeigt, dass Peptide und Proteine nur in äußerst geringen Konzentrationen, d.h. Stoffmengen von 10⁻¹² bis 10⁻⁹ M, von Zellen abgegeben werden. Da das Detektionslimit der gängigen Massenspektrometem zur Zeit bei 10⁻⁹ M liegt und für die massenspektrometrische Detektion die Substanzen hochrein vorliegen müssen, sind für die Anreicherung von Analyten in Zellkulturüberständen, z.B. Sekretionsmolekülen, effiziente Aufreinigungsschritte unabdingbar.

Aus diesem Grund liegt der Hauptaugenmerk der Proteomforschung momentan in der Präanalytik von niedrigkonzentrierten Substanzen. Eine Serie von verschieden aufeinander folgenden Aufreinigungsschritten führt aber bei einer geringen Proteinmenge durch unspezifische Bindungen an den zu verwendeten Gefäßwänden zu einem totalen Verlust an Protein. Ein zusätzliches Problem bei vielen Aufreinigungsschritten ergibt sich durch eine zunehmende Kontamination der Proben, die eine massenspektrometrische Analyse durch Störsignale unmöglich machen kann.

Deshalb konnten sekretorische Untersuchungen mit Zellen bisher nur mit sehr hohen Zellzahlen durchgeführt werden.

Experimente haben gezeigt, dass bei einer anfänglichen Zellzahl in der Größenordnung von 10⁷ Zellen und einer mehrtägigen Vermehrungsphase in einem serumhaltigen Medium und anschließender Inkubation in serumfreier Umgebung lediglich wenige mg Gesamtproteine erhalten werden. Bspw. wurde bei der Untersuchung von Hefezellen nach Abschluss der Zellkultur bei einer Zellzahl von 10⁹ Zellen eine Konzentration von 30 nM AIP-Protein in den Überständen gemessen. Solch große Mengen an Zellen stehen aber in der Regel nicht zur Verfügung. Bei der Untersuchung von Krebszellen, die aus Tumorgewebe durch Biopsie entnommen werden, können pro Stechzylinder maximal einige 10⁵ Zellen isoliert werden, wobei viele Zellen davon nicht Tumorzellen sind. Aufgrund der sehr geringen Konzentration der ausgeschütteten Substanzen ist es somit äußerst wichtig, die Sekretionsmoleküle in einem einzigen Aufreinigungsschritt effizient zu isolieren und der nachfolgenden Analytik ohne Umwege zuzuführen.

In den letzten 5 Jahren wurde versucht, als Screeningmethode für die von Zellen sekretierten Peptide und Proteine die SELDI-TOF-MS Technologie (**S**urface **E**nhanced **L**aser **D**esorption **I**onization **T**ime of **F**light **M**ass **S**pectrometry) einzusetzen. Hierzu werden auf einem Chip mit einer meist hydrophoben Oberfläche von der Größe von 3 mm² die zu untersuchenden Proben aufgegeben. Die auf dem Chip gebundenen Proteine werden anschließend durch Laserbeschuss ionisiert, massenspektrometrisch detektiert und in einem 2-dimensionalen Plot graphisch dargestellt. Durch die Anwendung dieser Methode konnten z.B. verschiedene Krebszelllinien miteinander verglichen werden. Ein limitierender Faktor ist jedoch die ungenügende Empfindlichkeit dieser Technologie. In der Regel liegt die Empfindlichkeitsgrenze für dieses System bei maximal 300 nM und aus diesem Grund wird die SELDI-TOF-MS-Technologie meist nur noch für das Profiling von Serumproben eingesetzt.

Eine Steigerung der Empfindlichkeit kann nur durch den Einsatz von Partikeln mit geringer Größe erfolgen, da dadurch eine größere Gesamtoberfläche für die Bindung von Protein zur Verfügung steht. Durch die Verwendung von magnetischen Partikeln als Trägersubstanzen für die selektierenden Proteine und Peptide ist eine Automatisierung und somit ein höherer Durchsatz an Proben möglich. Der Einsatz von magnetischen Partikeln, so genannte Beads, zur Isolierung von Proteinen wurde mit biotinylierten Antikörpern, welche an magnetische Streptavidinbeads gekoppelt waren, gezeigt und erfolgreich für die massenspektrometrische Untersuchung von Prostata Spezifischen Antigen (PSA) aus Seren heraus verwendet. Leider kann aber durch den Einsatz von magnetischen "Antikörper-Partikeln" jeweils nur ein einzelnes Protein isoliert werden.

Für die Bindung von möglichst vielen unterschiedlichen Peptiden oder Proteinen aus einer Lösung, wie Plasma, Serum, Urin oder Zellkulturmedium, haben sich Partikel mit hydrophoben Oberflächen durchgesetzt. Diese Partikel binden Proteine über hydrophobe Wechselwirkung und sind somit in der Lage, ein breites Spektrum an Peptiden bzw. Proteinen zu binden. So werden derartige magnetische Mikropartikel mit hydrophoben Oberflächen (RP-Beads) immer häufiger für die Entsalzung und Anreicherung von Peptiden und Proteinen z.B. aus Serum heraus angewendet. Diese so aus z.B. verschiedenen Seren aufgereinigten Peptide und Proteine werden nun normalerweise direkt auf ein MALDI-TOF MS Target aufgebracht, um z.B. Peptid- und Proteinprofile zu erstellen.

Serum besteht aus einer Vielzahl von Stoffen die in sehr unterschiedlichen Konzentrationen vorkommen. So kommt bspw. Albumin in einer Konzentration im mg/ml-Bereich vor, während z.B. Interleukine nur in einer Konzentration von ng/ml-Bereich vorkommt. Diese hochkonzentrierten Proteine wirken sich jedoch störend bei der Findung von niedrig konzentrierten Proteinen aus, wie sie von in einer Zellkultur wachsenden Zellen, z.B. Tumorzellen, in das Zellkulturmedium abgegeben werden. Derartige regulatorische Peptide und Proteine werden aber nur in sehr geringen Mengen abgegeben.

Hinzu kommt ferner, dass Zellen für ihr Wachstum in einem Zellkulturmedium als Zusatz im Allgemeinen noch fötales Kälberserum benötigen, kurz FKS. Dieser Zusatz liefert den Zellen zwar einerseits die notwendigen Wachstumsfaktoren, andererseits werden der Zellkultur auf diese Weise wieder zusätzliche Proteine mit sehr hohen Konzentrationen zugesetzt. Bei einer Bindung an hydrophobe Magnetbeads konkurrieren somit diese hochkonzentrierten Substanzen um die Bindungsstellen, wodurch niedrig konzentrierte Peptide und Proteine nur zu einem kleinen Bruchteil gebunden werden können.

Die mit Erfolg bei der Untersuchung von Serum bzw. Plasmaproben eingesetzten hydrophoben Magnetbeads sind somit für den Einsatz in einem Zellkulturmedium zur selektiven Separation von regulatorischen Peptiden und Proteinen, die von Tumorzellen in das Zellkulturmedium abgegeben werden, nur bedingt geeignet.

### Darstellung der Erfindung

Der Erfindung liegt die Aufgabe zugrunde ein Verfahren zur Aufreinigung wenigstens einer nachzuweisenden Zielsubstanz, die von Zellen in einem Zellkulturmedium abgegeben werden, bei dem dem Zellkulturmedium magnetische, an ihrer Oberfläche funktionalisierte Partikel, so genannte Beads, zugegeben werden, an deren Oberfläche sich die Zielsubstanz selektiv anlagert und durch Applikation eines Magnetfeldes die mit der Zielsubstanz behafteten Partikel aus dem Zellkulturmedium selektiert werden, derart weiterzubilden, dass eine selektive Separation der Zielsubstanz mit einer verbesserten Selektivität aus dem Zellkulturmedium möglich sein soll, wobei die Zielsubstanz mit nur geringer Menge bzw. Konzentration im Zellkulturmedium vorhanden ist.

Insbesondere soll das Aufreinigungsverfahren zur gezielten Extraktion von Substanzen dienen, die während der Inkubation von Zellen, insbesondere Tumorzellen, u.a. durch sekretorische Prozesse in dem Zellkulturmedium vorliegen und dies in geringer Konzentration von 10⁻¹² bis 10⁻⁷ M

Die Lösung der der Erfindung zugrunde liegenden Aufgabe ist im Anspruch 1 angegeben. Das lösungsgemäße Verfahren wird darüber hinaus durch die in den Unteransprüchen angegebenen Merkmalen sowie den in der Beschreibung insbesondere unter Bezugnahme auf die Ausführungsbeispiele entnehmbaren Merkmalen vorteilhaft weitergebildet.

Das lösungsgemäße Verfahren zur Aufreinigung wenigstens einer nachzuweisenden Zielsubstanz, vorzugsweise von regulatorischen Peptiden und Proteinen, die von Zellen, insbesondere von Tumorzellen, in einem Zellkulturmedium abgegeben werden, besteht darin, dass (a) die Zellkultur unter der Zuhilfenahme eines Serumersatzes durchgeführt wird und dass (b) dem Zellkulturmedium magnetische, an ihrer Oberfläche funktionalisierte Partikel, so genannte Beads, zugegeben werden, an deren Oberfläche sich die Zielsubstanz selektiv anlagert und durch Applikation eines Magnetfeldes die mit der Zielsubstanz behafteten Partikel aus dem Zellkulturmedium selektiert werden, zeichnet sich durch folgende Verfahrensschritte aus: (Zur besseren Verfolgbarkeit der einzelnen Hauptprozessschritte sind zu diese zugehörige Schlagworte fett editiert.)

**Bereitstellen eines Serumersatzes**, der aus einem natürlich vorkommenden Serum gewonnen wird und frei oder nahezu frei ist von niedermolekularen Substanzen, die eine Masse von kleiner gleich 60 kDa, 30 kDa, vorzugsweise kleiner gleich 10 kDa, aufweisen. So zeichnet sich der bereitzustellende Serumersatz insbesondere dadurch aus, dass er sich nicht, wie bei anderen Produkten, nachteilig auf das Zellwachstum auswirkt, aber vor allem keine störenden Substanzen mit einer Größe unterhalb 10 kDa enthält.

Als natürliches Serum bieten sich grundsätzliche tierische Seren an, vorzugsweise fötales Kälberserum, Rinder- oder Schafsserum, um nur einige zu nennen. Auch kann menschliches Serum verwendet werden.

Zur Aufbereitung und Bereitstellung des Serumersatzes wird in einer bevorzugten Ausführungsvariante das natürliche Serum zunächst unter Einsatz von Ultrafiltrationseinheiten zentrifugiert, so dass ein Überstand, das so genannte Retentat, und ein Permeat erhalten wird. Im Letzteren, dem Permeat, befinden sich je nach verwendeter Filtergröße Substanzen mit Partikelgrößen kleiner der jeweils verwendeten Filterporengröße. Hierbei kommen Membranen mit geeignet gewählten Porengrößen bzw. Ausschlussgrenzen zwischen 3 KDa und 100 kDa zum Einsatz. Um einen möglichst an Kleinstpartikeln befreiten Serumüberstand zu erhalten, wird der im Rahmen des ersten Zentrifugationsdurchgang erhaltene Überstand mit einem Nährmedium versetzt und einer erneuten Zentrifugation unterzogen. Dieser auch als Waschvorgang zu bezeichnende Vorgang wird in vorteilhafter Weise mehrmals wiederholt. Als Waschlösung, das dem jeweils erhaltenen Überstand beizumischen ist, bieten sich Nährmedien, wie Dulbecco's Modified Eagle Medium, kurz DMEM, IMDM, IMEM, ERDF, RPMI1640 oder Puffer, wie PBS, MES, TRIS, etc. an.

Nach dem Waschvorgang erfolgt im Weiteren ein Rückzentrifugieren des erhaltenen Überstandes bzw. des Retentats, wobei das Gefäß in dem der Überstand enthalten ist lediglich umgedreht wird und abermals einer Zentrifugation unterzogen wird, wobei nun das Retentat aus der Zentrifugationseinheit zur weiteren Verarbeitung in ein Auffanggefäß herausbefördert und mit dem jeweils eingesetzten Nährmedium oder Puffer versetzt wird, vorzugsweise durch Zugabe von soviel an Nährmedium, dass das ursprünglich eingesetzte Volumen erreicht wird.

Der auf die vorstehende Weise erhaltene, so bezeichnete Serumüberstand wird zu Zwecken einer weiteren Reinigung sowie Sterilisation einer sterilen Filtration unterzogen, vorzugsweise unter Einsatz eines 0,2 µm-Filters, und anschließend als der dem lösungsgemäßen Verfahren bereitzustellende Serumersatz in praktikable Mengen von bspw. 500 µl-Aliquots portioniert und für eine künftige Verwendung bei - 20°C bis - 80°C eingefroren.

**Beimischen** des vorstehend beschriebenen Serumersatzes zu dem mit den Zellen versetzen Zellkulturmedium, für das gleichsam dem vorstehend beschriebenen Nährmedium Dulbecco's Modified Eagle Medium, kurz DMEM, IMDM, IMEM, ERDF oder RPMI1640 geeignet ist. Das Beimischen des Serumersatzes erfolgt mit einem Volumenanteil von 1-10%, in dem die Zellen über mehrere Tage, d.h. 1 bis 14 Tage, **inkubiert** werden. Ein während der Inkubation des Gemisches aus Zellkuturmedium, Zellen und Serumersatz mit Substanzen angereicherter Zellkulturüberstand, wird im Weiteren **separiert** und zu Zwecken eines Transportes oder zu Zwecken der Aufbewahrung in entsprechend konfektionierte Behältnisse, so genannte Vials, abgefüllt und gegebenenfalls eingefroren.

Zur weiteren Verarbeitung bzw. Aufreinigung der sich innerhalb des Zellkulturüberstandes befindlichen Substanzen wird der Zellkulturüberstand nach entsprechendem Auftauen einer **Zentrifugation** mittels einer Ultrafiltrationseinheit unterzogen. Hierbei finden Membranen mit geeignet gewählten Porengrößen bzw. Ausschlußgrenzen zwischen 3 kDa bis 60 kDa bevorzugt Einsatz. Das durch die **Filtration** gewonnene Filtrat wird im Weiteren durch Behandlung mit geeignet funktionalisierten Magnetbeads aufgereinigt.

Zur erfolgreichen Anwendung des lösungsgemäßen Verfahrens sind besonders funktionalisierte, magnetische Partikel, kurz Beads oder Magnetbeads, **bereitzustellen,** die im Vergleich zu bislang kommerziell erhältlichen Beads über eine höhere Bindekapazität und eine neue Methode zur Bindung von Substanzen verfügen, die sich durch eine hohe Anreicherung der niedrig konzentrierten Substanzen auszeichnet. So weisen die neuartigen Magnetbeads an ihren funktionalisierten Oberflächen eine Vielzahl an Dendrimeren mit jeweils bis zu 10 Verzweigungen auf, d.h. zehn Generationen, wobei die Endstellen der jeweils letzten Generation der Dendrimere modifiziert werden.

Derartig neue Magnetbeads sind beispielsweise in folgender Weise herstellbar bzw. konfektionierbar:
Zunächst gilt es die magnetischen Partikel bereitzustellen, die es kommerziell in unterschiedlichen Partikelgrößen im Nano- oder Mikro-Bereich, d.h. ein oder mehrere nm bzw. µm, zu erhalten gibt, an deren Oberflächen beispielsweise Aminogruppen appliziert sind. Zur weiteren Funktionalisierung der Aminobeadoberfläche werden die Beads in ein organisches Lösungsmittel gegeben, dem Methylacrylat zugegeben worden ist. Nach einer Einwirkzeit, die vorzugsweise 2 bis 48 Stunden von Dauer ist,
werden die Beads aus der Reaktionsflüssigkeit abgetrennt und mit frischem Lösungsmittel gewaschen. Anschließend werden die so vorbereiteten Beads zur Ausbildung einer ersten Dendrimergeneration (G1-Beads) an ihrer Oberfläche in eine organische Reaktionsflüssigkeit, der Ethylendiamin zugegeben worden ist. In dieser Umgebung reagieren die Beads weitere 2 bis 48 Stunden, wobei der Reaktionsvorgang durch Schütteln des Reaktionsbades unterstützt werden kann.
Anschließend werden die Beads aus der Reaktionsflüssigkeit erneut, vorzugsweise unter Verwendung eines Magneten separiert und gewaschen.
Um nun eine mehrfache Dendrimerausbildung zu erlangen, auf die letztlich die überaus hohe Bindefähigkeit der neuartig funktionalisierten Beads zurückzuführen ist, gilt es den vorstehend beschriebene Vorgang zur Ausbildung der ersten Dendrimergeneration G1 bis zu neun mal zu wiederholen.

Liegen nach entsprechender Prozessierung Dendrimer-Beads vor so werden diese in einem Lösungsmittel unter Hinzufügen von aktivierten Alkyl- (C₁ - C₃₀) oder Aromatenderivaten, z.B. Monoaromaten oder Polyaromaten, suspendiert, um letztlich an den Dendrimerkettenendstellen eine Modifizierung mit hydrophoben Derivaten zu erreichen, wodurch die Beads über hydrophobe Eigenschaften verfügen. Man bezeichnet diese funktionalisierten Beads deshalb auch als dendritische Reversed Phase Beads (G1-RP-Beads bis G10-RP-Beads).

Zur Bereitstellung werden die Beads aus der Reaktionssuspension separiert, abermals gewaschen und in destilliertem Wasser oder Puffer, wie PBS, MES, TRIS etc. bevorratet.

Die auf die vorstehende Weise erhaltenen, so genannten Dendrimer-RP-Beads werden zu Zwecken der weiteren Aufreinigung und Separierung der sich innerhalb des steril gefilterten Zellkulturüberstandes befindlichen Substanzen, u.a. von den Zellen sekretierte regulatorische Peptide und Proteine dem nach der Filtration erhaltenen Filtrats unter Zugabe einer Pufferlösung, **beigemischt.**

Anschließend erfolgt eine **Inkubation** mit einer Verweilzeit von 10 bis 120 Minuten bei Raumtemperatur. Hierbei erfolgt die Bindung der in dem Zellkulturüberstand befindlichen Substanzen an die modifizierten Endstellen der Dendrimerstrukturen. Anschließend erfolgt die **magnetische Selektion** der magnetischen Beads aus dem Gemisch und eine **Elution** der an der dendritischen Struktur gebundenen nachzuweisenden Substanzen, vornehmlich die von den Zellen sekretierten Substanzen, allen voran die regulatorischen Peptide und Proteine. Selbstverständlich können je nach Funktionalisierung der Magnetbeads auch weitere Substanzen gezielt selektiert werden, so bspw. Metabolite, Steroide etc.

Der Kerngedanke des lösungsgemäßen Aufreinigungsverfahren umfasst im Wesentlichen zwei vorteilhaft zusammenwirkende Grundgedanken, nämlich zum einen die Bereitstellung eines Zellkulturzusatzes bzw. Serumersatzes, in dem von vornherein keine störende Kleinsubstanzen enthalten sind, die möglicherweise bei der nachfolgenden Analyse in Konkurrenz mit den eigentlich nachzuweisenden Zielsubstanzen treten, zum anderen neuartiger Magnetbeads mit einer dendritischen Struktur und einer RP-Phase (reversed phase), wobei die dendritische Struktur bis zu zehn Verzweigungsstellen aufweist und somit die Anzahl der für eine Bindung von nachzuweisenden Zielsubstanzen optimiert.

Durch das lösungsgemäße Verfahren können weit mehr Substanzen isoliert werden als bei der Verwendung von bisher im Einsatz befindlichem normalem Serum, wie bspw. fötales Kälberserum. Durch Verwendung der neuartigen Beads kann im Vergleich zu kommerziell erhältlichen eine 10 bis 100-fache Steigerung der Sensitivität aus bsp. Zellkulturübertständen erreicht werden. Zusammengefasst wird bei der Verwendung der Beads und des Serumersatzes eine bis zu 1000-fach höhere Empfindlichkeit erzielt, wie bei der Anwendung von normalem Serum und kommerziell erhältlichen Beads.

Im Weiteren werden einige konkrete Ausführungsbeispiele zur Verfahrensführung dargestellt:

### Beispiel 1: Herstellung eines Serumersatzes

- (a): Niedermolekulare Substanzen aus fötalem Kälberserum oder aus humanem Serum werden durch Zentrifugation und Waschen mit Dulbeccos Eagle Modified Medium entfernt. Hierbei erfolgt die Zentrifugation über eine Filtrationsmembran, die je nach Bedarf eine Porengröße zwischen 3 kDa und 100 kDa haben kann. Der Zentrifugationsvorgang wird eine Stunde durchgeführt. Bei Bedarf kann das Serum mit Proteaseinhibitoren (PMSF, EDTA, Proteaseinhibitoren in Form von einzelnen Inhibitoren oder eines Cocktails etc.) vor der Aufarbeitung versetzt werden.
- (b): Der im Wege der Zentrifugation erhaltene Überstand wird mit Dulbeccos Medium Eagle Medium (DMEM) versetzt und wieder 1 Stunde zentrifugiert (Reinigung des Serums).
- (c): Der Zentrifugations- und Waschschritt werden insgesamt 2-5x durchgeführt.
- (d): Der erhaltene Überstand wird nun in ein Gefäß rückzentrifugiert und mit DMEM auf das ursprünglich eingesetzte Volumen eingestellt.
- (e): Der erhaltene Serumersatz (auch Serumüberstand genannt) wird über einen 0.2µm-Filter steril filtriert.
- (f): Der Serumersatz wird zu 500 µl-Aliquots portioniert und bei -20°C bis zur Verwendung eingefroren.

### Beispiel 2: Herstellung von modifizierten Magnetbeads

Ausgehend von der Aminoberfläche der Beads wird über eine Verzweigungsreaktion an jeder Aminogruppe eine dendritische Struktur aufgebaut. Hierbei wird das Prinzip zur Synthese von Starburstmolekülen zugrunde gelegt, bis eine bis zu zehnfache-Verzweigung in der dendritischen Struktur zum Erhalt so genannter G1- bis G10-Beads erreicht wird. Am Schluss erfolgt die Modifizierung der endständigen NH₂-Gruppen der Dendrimer-Beads u. a. mittels Mikrowellensynthese mit unterschiedlich langen Alkylketten (C₁-C₃₀) über eine aktivierte Gruppe.
- (a): Bereitstellen von 10 -2000 mg Aminobeads und in einem Behältnis mehrmals mit 10 bis 100 ml MeOH waschen.
- (b): Beads in 10 -100 ml MeOH aufnehmen und 10 - 100 ml kaltes Methylacrylat zugegeben. Auf einem Schüttler 2 - 48 Stunden bei Raumtemperatur (RT) reagieren lassen.
- (c): Abtrennen der Beads von der Reaktionsflüssigkeit durch einen Magneten und Beads 3x mit jeweils 10 - 200 ml MeOH waschen.
- (d): Zugabe von 10 - 100 ml MeOH und langsame Zugabe von 10 - 100 ml kaltem Ethylendiamin. Bei Raumtemperatur 2 - 48 h unter Schütteln regieren lassen; (G1 -Beads).
- (e): siehe Schritt (c), d.h. Abtrennen der Beads von der Reaktionsflüssigkeit durch einen Magneten und Beads 3x mit jeweils 10 -100 ml MeOH waschen.
- (f): N-malige Wiederholung der Schritte (b) - (e), wobei n= 0 bis 9 ist.
- (g): Waschen der Dendrimer-Beads jeweils 3x mit 10 -100 ml MeOH und 5x mit 10 -100 ml halogeniertem Kohlenwasserstoff,
- (h): Dendrimer-Beads in 50 -100 ml halogeniertem Lösungsmittel suspendieren und langsames Zutropfen von 5 -10 ml eines aktivierten Alkylderivates, z.B. C1-C30 oder Aromatenderivates. Reaktion für 10 bis 120 Minuten bei RT im Ultraschallbad und/oder unter Erhitzen bis maximal 38°C oder mittels Mikrowellensynthese.
- (i): Modifizierte Dendrimer-Beads jeweils 5 x mit 10 - 100 ml MeOH und 5 x mit 10 - 100 ml ultrareinem Wasser waschen.
- (j): Aufnahme der Dendrimer-Beads in ultrareinem Wasser und Lagerung bei +4°C.

Alternativ ist es anstelle des Verfahrensschrittes (h) ebenso möglich die Dendrimer-Beads anderweitig an den Endstellen ihrer Dendrimerketten zu modifizieren, bspw. durch Hinzufügung von Antikörpern. Hierzu werden die vorprozessierten dendritischen Aminobeads (G1-G10) in einem Puffer mit einem bifunktionalem Linker für Aminogruppen zur Reaktion gebracht. Als Linker eignet sich vorzugsweise Glutardialdehyd, Bis (Sulfosuccinimidyl) suberate, Succinimidylsuberate, Dimethyl Pimelidate oder bifunktionelle PEG-Linker. Anschließend erfolgt ein Waschen der Beads vorzugsweise in einem Phosphatpuffer. Schließlich werden dem Puffer Antikörper in einer Konzentration von 1 mg/ml bis 50 mg/ml Puffer zugegeben. Nach einer Inkubation von ca. 1 -14 h bei RT oder +4°C werden die mit Antikörpern versehenen Beads gewaschen und in einem Puffer zur weiteren Verwendung bei etwa +4°C bevorratet.

Beispiel 3: Durchführung einer Aufreinigung von sekretierten Substanzen von in einem Zellkulturmedium vorliegenden Tumorzellen unter Verwendung des in Beispiel 1 gewonnenen Serumersatzes sowie der in Beispiel 2 modifizierten magnetischen Partikel

Die Tumorzellen werden je nach Größe des Experiments in Dulbeccos Modified Eagle Medium (DMEM) und 1-10% Serumersatz, der aus fötalem Kälberserum (FKS) erhalten worden ist, in verschiedenen Behältnissen eingesät und über mehrere Tage bei 37°C und 5% CO₂Anreicherung inkubiert. Der Zellkulturüberstand, d.h. DMEM + 1-10% FKS-Serumersatz, kurz ZKÜ genannt, wird in vorher behandelte Vials abgefüllt und zum Transport bei -20°C eingefroren.

Vor der Aufreinigung mit den modifizierten Dendrimer-Beads (G1-G10) wird der aufgetaute ZKÜ über eine Membranfiltrationseinheit filtriert. Das Filtrat wird im Weiteren mittels der modifizierten Dendrimer-Beads (G1-G10) aufgereinigt und konzentriert. Anschließend erfolgt die Elution und weitere Aufarbeitung.

Im Einzelnen:
- (a): Zellen in DMEM und FKS-Überstand aufnehmen und je nach Ansatz (Volumen) in geeignetem Zellkulturgefäß bei 37°C und 5% CO₂ für 3 Tage inkubieren.
- (b): Verwerfen des ZKÜ und vorsichtiges Waschen der Zellen mit jeweils 10 ml PBS, bei pH 7.2 oder DMEM, den Waschvorgang 2-5x wiederholen.
- (c): Zugabe der erforderlichen Menge DMEM + 1-10% FKS-Serumüberstand und Inkubation der Zellen über die erforderliche Zeit (1- 14 Tage). Nach erfolgter Inkubation ZKÜ's in vorbehandelte Vials (1 ml) geben und bei -20°C oder bei -80°C einfrieren.
- (d): Auftauen der eingefroren ZKÜ's und jeweils 500 µl in eine Filtrationseinheit überführen.
- (e): Zentrifugation der ZKÜ für 1 h bei 12000 rpm und Filtrat auffangen.
- (f): In der Zwischenzeit Vorbereitung der Beads für die Inkubation. 80 µl der modifizierten Dendrimer-Beads (G1- G10) in 450 µl Puffer bei pH 7.2 aufnehmen und kurz suspendieren. Danach Abtrennen der Beads über einen Magneten.
- (g): Zugabe von 450 µl eines Puffers mit pH 7.2 und Beads suspendieren. Anschließend 450 µl des von der ZKÜ-Zentrifugation erhaltenen Filtrats mit einer Pipette zugeben und 10 x auf und ab pipettieren (Beads vollständig suspendieren).
- (h): Inkubation für 10 -120 min bei Raumtemperatur.
- (i): Lösung nach magnetischer Separation verwerfen und magnetische Dendrimer-Beads (G1-G10) 3 x mit jeweils 500 µl Puffer waschen.
- (j): Zugabe von 20 µl Acetonitril:TFA 1:1, v:v und mischen der Beadlösung durch 10-maliges auf- und abpipettieren (Elution).
- (k): Inkubation für 1 - 120 min bei Raumtemperatur.
- (l): Abtrennung der magnetischen Partikel und Elutionslösung für weitere Aufarbeitung separieren.

Beispiel 4: Fünf alternative Beispiele zur weiteren Untersuchung der mit den Zielsubstanzen versehenen magnetischen Beads, wie sie nach dem lösungsgemäßen Aufreinigungsverfahren gewonnen werden.

Erste Alternative:
Die mit den Zielsubstanzen beaufschlagten magnetischen Beads werden zu einem nicht reduzierenden oder reduzierenden Gelelektrophoreseprobenpuffer, bestehend aus TRIS, Harnstoff. Thioharnstoff, LDS, SDS oder CHAPS etc., zugegeben.
Das sich dabei bildende Gemisch wird vorzugsweise auf 60°C für 5 - 30 min erhitzt, wodurch sich die Zielsubstanzen von den Beads lösen. Im Weiteren werden die magnetischen Beads mittels eines Magneten aus dem Gemisch abgetrennt und der verbleibende Gelelektrophoreseprobenpuffer wird mit den Zielsubstanzen abpipettiert und in Geltaschen eines Elektrophoresegels (SDS-Gel, Native-Gel, 2-D-Gel) aufgegeben und nachfolgend einer elektrophoretischen Auftrennung unterzogen.

Zweite Alternative (gilt nur für mit Antikörper gekoppelten Dendrimer-Beads (G1-G10):
Die an den Antikörperpartikeln gebundenen Zielsubstanzen werden durch Zugabe einer Carbonsäure oder eines chaotropen Hochsalzpuffers eluiert. Das sich im Wege der Elution ergebende Carbonsäureeluat wird einrotiert (Speed-Vac) und in einer Pufferlösung aufgenommen. Schließlich unterliegen die in der Pufferlösung enthaltenen Zielsubstanzen einem enzymatischen Verdau. Die verdauten Zielsubstanzen werden anschließend einer massenspektrometrischen Identifizierung über Peptide Mass Fingerprint (PMF) oder MS/MS-Spektren unterzogen.

Dritte Alternative:
Die mit einer oder mehreren Zielsubstanzen beaufschlagten magnetischen Beads werden gewaschen und anschließend einer Pufferlösung mit einer Protease zum Verdau der Zielsubstanzen zugeführt. Die verdauten Zielsubstanzen werden anschließend einer massenspektrometrischen Identifizierung über Peptide Mass Fingerprint (PMF) oder MS/MS-Spektren unterzogen. Desweiteren werden die magnetischen Beads abermals gewaschen und anschließend einem Gemisch aus einem organischen Lösungsmittel und/oder Carbonsäuren zugegeben, zum Erhalt eines Eluats. Letztlich wird das Eluat wieder einer massenspektrometrischen Messung unterzogen.

Vierte Alternative:
Die an den Antikörpern gebundenen Zielsubstanzen werden durch Zugabe einer Carbonsäure oder eines Hochsalzpuffers eluiert. Zu den Eluaten werden die dendritischen reversed-phase Beads gegeben. Nach einer Inkubation von 1 - 60 Minuten, werden die Beads mit Hilfe eines Magneten separiert und anschließend mehrmals mit einem Puffer gewaschen.
Anschließend erfolgt entweder der in Alternative 1 aufgezeigte Weg für eine Gelelektrophoretische Auftrennung oder der in Alternative 3 aufgezeigte Weg für eine Erzeugung von proteolytischen Peptide, die anschließend über MS- oder MS/MS-Spektren detektiert und aufgeklärt werden.

Fünfte Alternative:
Zellen, wie z.B. Tumorzellen, werden je nach Experiment in verschiedene Behältnisse eingesät und für mehrere Tage bei 37°C und 5% CO₂-Gehalt inkubiert.
   Danach erfolgt ein Waschen der Zellen mit Zellkulturmedium oder Puffer.
   Anschließend erfolgt die Zugabe von Zellkulturmedium ohne Serum und eine nochmalige Inkubation von 0 - 72 h. Die jeweiligen Zellkulturüberstände werden abgefüllt und gegebenenfalls bei -20°C eingefroren.

Die Bindung der Substanzen an die modifizierten Dendrimerbeads erfolgt durch Zugabe derselben zu den aufgetauten Zellkulturüberständen . Anschließend erfolgt ein Waschen der Beads und diese werden wie in Alternative 1 aufgezeigt, einer Gelelektrophorese unterzogen oder wie in Alternative 3 aufgezeigt einem Verdau mit einer Protease unterzogen und die verdauten Substanzen einer MS bzw. MS/MS-Analyse unterzogen.

Die vorstehend beschriebenen funktionalisieren Magnetbeads stellen als solche ein Produkt dar, das als sensorisch wirkende Substanz grundsätzlich für eine Vielzahl unterschiedlicher Einsatzwecke verwendbar ist. Ein möglicher Einsatz ist die vorstehend beschriebene Aufreinigung von Zielsubstanzen. Zur Herstellung eines solchen Produktes in allgemeinen Form sind folgende Maßnahmen zu treffen:
- a): Bereitstellen von magnetischen Partikeln, kurz Beads, mit einer Partikelgröße im Nano- und/oder Mikro-Bereich, d.h. ein oder mehrere nm oder µm, und mit an deren Oberflächen applizierte funktionelle Gruppe, wie z.B. Aminogruppen,
- b): Reagieren der Beads in einem Verzweigungsreagenz, wie z.B. Methylacrylat enthaltenden organischen Lösungsmittel,
- c): Abtrennen der Beads aus dem organischen Lösungsmittel und Waschen der Beads mit einem organischen Lösungsmittel,
- d): Reagieren der Beads mit einem Verbinder, wie z.B. Ethylendiamin, enthaltenden organischen Flüssigkeit,
- e): Abtrennen der Beads aus der organischen Flüssigkeit und Waschen der Beads,
- f): n - maliges Wiederholen der Schritte b) bis e)
- g): Suspendieren der Beads in einer mit wenigstens einem Derivat enthaltenden Lösung, und
- h): Abtrennen und Waschen der Beads und Bereitstellen der derivatisierten Beads in Wasser oder pufferhaltiger Lösung.

Vorzugsweise wird n = 0 bis 9 gewählt. Als Reaktionsflüssigkeit eignet sich vor allem Alkohol. Damit die Beads reversed-phase Eigenschaften annehmen, werden die Beads mit einer chemischen oder proteinogenen Gruppe, wie Alkyl-(C₁-C₃₀) oder Aromatgruppen derivatisiert. Als Grundkörper für die Beads eignen sich stabile magnetische Partikel, die eine Partikelgröße von 10 nm -10 µm aufweisen.

Die auf den magnetischen Partikeln zu Zwecken ihrer Funktionalisierung applizierte Gruppe ist vorzugsweise aus wenigstens einem Stoff der nachfolgenden Stoffgruppen aufgebaut: Primäre und sekundäre Amino-, prim., sek. und unsym. Hydrazin-, Azid-, Phosphin-, Aldehyd-, Polyaldeyhd-, Carbonsäure-, Carbonsäureester-, Cyanat-, Isocyanat-, Thiocyanat-, Hydroxy-, Thiol-, Imino-, Hydrazid-, Piperidin-, Azomethin-, Semicarbazon-, Hydrazon-, Cyanbromid-, Tosyl-, Epoxid-,Cyanursäurechlorid-, Cyanursäureester,-gruppen. Diamino-, Triamino-,Tetraaminoheterocyclen.

Die vorstehend beschriebenen funktionalisierten Beads werden ferner mit Verzweigungsreagenzien, die in einem Lösungsmittel bereitgestellt werden, zur Reaktion gebracht, wobei die Verzweigungsreagenzien aus wenigstens eine der nachfolgenden Gruppe ausgewählt sind:
Melamin, Diamino-, Triamino-, Tetraaminoheterocyclen, Triepoxide, Tetraepoxide, Diallylamine, Methylacrylate, Triacrylate, Tetraacrylate, Tris(hydroxymethylamin), Oxazine, Oxetane, Diethanolamine.

Anschließend werden die mit dem Verzweigungsreagenz beaufschlagten magnetischen Partikel mit einem bi- oder trifunktionalen Linker zur Reaktion gebracht werden, wobei der der Linker aus einer der nachfolgenden Stoffzusammenstellung ausgewählt wird: Lysine, Diaminoalkane, Diamino-, Triamino-, Tetraaminoheterocyclen, Diaminoethylenglycole, Piperazin, Allylamine, Triepoxide, Tetraepoxide, Tris(hydroxymethylamin).

Schließlich werden nach n-maligem Wiederholen der vorstehend genannten Verfahrensschritte b) bis e) die magnetischen Partikel mit nachfolgenden chemischen Gruppen als Endgruppe oder mit Peptiden, Proteinen, Glycanen oder Glycoproteinen aller Art derivatisiert:
n-Alkyl-, sek-Alkyl-, tert-Alkylgruppen, unsubstituirte und substituirte Aryl-, unsubstituirte und substituirte Acyl-, primäre, sekundäre und tertiäre Amino-, prim., sek. und unsym. Hydrazin-, Azid-, Aldehyd-, Phosphin-, Polyaldeyhd-, Carbonsäure-, Carbonsäureester-, Carbonsäurehalogenid-, Carbonsäureanhydrid-, Cyanat-, Isocyanat-, Thiocyanat-, Hydroxy-, Thiol-, Sulfid-, Sulfit-, Sulfat-, Imino-, Hydrazid-, Piperidin-, Azomethin-, Semicarbazon-, Hydrazon-, Hydroxamsäure-, Amidrazon-, Amidin-, Cyanbromid-, Tosyl-, Epoxid-,Cyanursäurechlorid-Cyanursäureester-gruppen, unsubstituierte und substituierte Carbodiimide, N-Hydroxysuccinimidester, Ethylenglycole, unsubstituierte und substituierte Arylalkane, Arylalkene, Heterocyclische Verbindungen, Mono- und Polycyclische Verbindungen, Die Anzahl der Kohlenstoffe beträgt hierbei 2 bis 100.

## Patentansprüche

1. Verfahren zur Aufreinigung wenigstens einer nachzuweisenden Zielsubstanz, die während einer Zellkultivierung in einem Zellkulturmedium auftritt oder gebildet wird, bei dem dem Zellkulturmedium magnetische, an ihrer Oberfläche funktionalisierte Partikel, so genannte Beads, zugegeben werden, an deren Oberfläche sich die Zielsubstanz selektiv anlagert und durch Applikation eines Magnetfeldes die mit der Zielsubstanz behafteten Partikel aus dem Zellkulturmedium selektiert werden,
**gekennzeichnet durch** folgende Verfahrensschritte:
- Bereitstellen eines Serumersatzes, der aus einem natürlich vorkommenden Serum gewonnen wird und frei oder nahezu frei ist von niedermolekularen Substanzen, die eine Masse von kleiner gleich 60 kDa, insbesondere kleiner gleich 10 kDa, aufweisen,
- Beimischen des Serumersatzes zum Zellkulturmedium, in dem die Zellen bereits enthalten sind oder in das die Zellen beigegeben werden,
- Inkubation der Zellen in dem mit Serumersatz angereicherten Zellkulturmedium,
- Separation wenigstens eines Teils eines sich während der Inkubation einstellenden Zellkulturüberstandes,
- Filtration des Zellkulturüberstandes im Wege einer Ultrafiltration zum Erhalt eines Retentats,
- Bereitstellen der Beads derart, dass die funktionalisierte Oberfläche der Beads eine Vielzahl an Dendrimeren mit jeweils bis zu 10 Verzweigungen, d.h. zehn Generationen, aufweist, wobei die Endstellen der jeweils letzten Generation der Dendrimere modifiziert werden,
- Beimischen der Beads und einer Pufferlösung zu dem Retentat unter der Ausbildung eines Gemisches,
- Inkubation und Bindung der im Retentat enthaltenen Zielsubstanzen an die Beads und magnetische Selektion der magnetischen Beads aus dem Gemisch.

2. Verfahren nach Anspruch 1,
**dadurch gekennzeichnet, dass** der Serumersatz als Retentat im Wege einer Ultrafiltration aus einem natürlichem Blutserum eines Tieres oder Menschen gewonnen wird, und dass das Retentat mittels eines Nährmediums oder Puffer gewaschen wird, wobei als Waschlösung vorzugsweise Nährmedium, Dulbecco's Modified Eagle Medium (DMEM), IMDM, IMEM, ERDF oder RPMI1640 verwendet wird.

3. Verfahren nach einem der Ansprüche 1 oder 2,
**dadurch gekennzeichnet, dass** der Serumerssatz in folgender Weise gewonnen und bereitgestellt wird:
a) Zentrifugieren von natürlichem Serum über eine Ultrafiltrationseinheit zum Erhalt eines Überstandes, dem so genanten Retentat, und einem Permeat,
b) Zusetzen des Retentats mit einem Nährmedium und anschließendem Zentrifugieren gemäß Schritt a)
c) n-maliges Wiederholen des Schrittes b)
d) Zugeben von Nährmedium an das erhaltene Retentat zum Erhalt eines so genannten Serumüberstandes und
e) Filtration des Serumüberstandes zum Erhalt eines dem Serumersatz entsprechenden Permeats, wobei bei der Filtration des Serumüberstandes vorzugsweise eine Sterilfiltration durchgeführt wird, oder wobei das natürliche Serum vor dem Schritt a) mit wenigstens einem reversiblen oder irreversiblen Proteaseinhibitor versetzt wird.

4. Verfahren nach Anspruch 3,
**dadurch gekennzeichnet, dass** als Proteaseinhibitor Phenylmethylsulfonylfluorid (PMSF) und/oder Ethylendiamintetraessigsäure (EDTA) und/oder proteinogene Inhibitoren einzeln oder als Proteaseinhibitoren Cocktail dem Serum zugesetzt werden.

5. Verfahren nach Anspruch 3,
**dadurch gekennzeichnet, dass** das Zentrifugieren gemäß der Schritte a) und b) wenigstens eine Stunde bei Raumtemperatur bis hinab zu +4°C durchgeführt wird, oder
**dadurch gekennzeichnet, dass** der Schritt b) n gleich 2-5 mal wiederholt wird, oder **dadurch gekennzeichnet, dass** die Filtration gemäß Schritt e) mit einem 0,2 µm Sterilfilter durchgeführt wird.

6. Verfahren nach Anspruch 1,
**dadurch gekennzeichnet, dass** der Zellkulturüberstand zeitlich vor der Filtration bei -20°C bis -80° C eingefroren wird,
dass der eingefrorene Zellkulturüberstand aufgetaut und,
dass die Filtration im Wege einer Zentrifugation unter Verwendung einer Ultrafiltrationseinheit durchgeführt wird, oder
**dadurch gekennzeichnet, dass** bei dem Beimischen der Beads sowie des Retentats eine antichaotrope Pufferlösung verwendet wird, oder
**dadurch gekennzeichnet, dass** nach der Inkubation, der Bindung der im Retentat enthaltenden Zielsubstanz an die Beads und magnetischen Selektion der Beads die mit den Zielsubstanzen versehenen Beads mit einer Niedrigsalzpufferlösung gewaschen werden, und
dass anschließend die wenigstens eine Zielsubstanz von den Beads in einem Gemisch aus wenigstens einer organischen Komponente und/oder einer Säure eluiert wird,
wobei als organische Substanz vorzugsweise Alkohol oder Acetonitril und als Säure vorzugsweise Carbonsäure oder Mono-, Di-, Trihalogencarbonsäure verwendet werden.

7. Verfahren nach einem der Ansprüche 1 bis 6,
**dadurch gekennzeichnet, dass** als Zellkulturmedium Dulbecco's Modified Eagle Medium (DMEM), IMDM, IMEM, ERDF oder RPMI1640 verwendet wird, und/oder **dadurch gekennzeichnet, dass** beim Beimischen des Serumersatzes zum Zellkulturmedium, 1 - 10 Vol.% Serumersatz in das Zellkulturmedium beigegeben wird, und/oder
**dadurch gekennzeichnet, dass** zum Bereitstellen der Beads folgende Schritte durchgeführt werden:
a) Bereitstellen von magnetischen Partikeln, kurz Beads, mit einer Partikelgröße im Nano- und/oder Mikro-Bereich, d.h. ein oder mehrere nm oder µm, und mit an deren Oberflächen applizierte Aminogruppen,
b) Reagieren der Beads in einem Methylacrylat enthaltenden organischen Lösungsmittel,
c) Abtrennen der Beads aus dem organischen Lösungsmittel und Waschen der Beads mit einem organischen Lösungsmittel,
d) Reagieren der Beads in einer Ethylendiamin enthaltenden organischen Flüssigkeit,
e) Abtrennen der Beads aus der organischen Flüssigkeit und Waschen der Beads,
f) n - maliges Wiederholen der Schritte b) bis e)
g) Suspendieren der Beads in einer mit wenigstens einer funktionalisierenden Substanz enthaltenden Lösung, und
h) Abtrennen und Waschen der Beads und Bereitstellen der funktionalisierten Beads in Wasser.

8. Verfahren nach Anspruch 7,
**dadurch gekennzeichnet, dass** als n = 0 bis 9 gewählt wird, und/oder **dadurch gekennzeichnet, dass** als Reaktionsflüssigkeit Alkohol, kurz R-OH, verwendet wird, mit R = C₁ bis C₆, und/oder
**dadurch gekennzeichnet, dass** das Suspendieren im Rahmen eines Ultraschallbades und/oder unter Erhitzen auf maximal 36°C und/oder im Rahmen einer Mikrowellensynthese durchgeführt wird, und/oder
**dadurch gekennzeichnet, dass** als die wenigstens eine funktionalisierende Substanz enthaltende Lösung halogenhaltige Lösungsmittel unter Hinzufügen von aktivierten Alkylderivaten (C₁ - C₃₀) verwendet werden, wobei als aktivierte Gruppen Halogene, insbesondere Cl, Br, J, Cyanate, Iso-Cyanate oder Isothiocyanate eingesetzt werden, oder
**dadurch gekennzeichnet, dass** nach dem Verfahrensschritt f) die Beads mit einem bifunktionalem Linker für Aminogruppen zur Reaktion gebracht werden,
dass die Beads in einem Puffer gewaschen werden,
und dass als die wenigstens eine funktionalisierende Substanz enthaltende Lösung Antikörper in einer Konzentration von c = 1 mg/ml bis 50 mg/ml in einer Pufferlösung verwendet wird,
wobei als bifunktionaler Linker vorzugsweise Glutardialdehyd, Bissulfosuccinimidylsuberate (BS3), Disuccinimidysuberate (DSS), Dimethyl Pimelidate (DMP) oder bifunktionale PEG-Linker verwendet wird,
oder wobei als Puffer zum Waschen vorzugsweise Phosphat- oder Carbonatpuffer verwendet wird.

9. Verfahren nach einem der Ansprüche 1 bis 8,
**dadurch gekennzeichnet, dass** den mit der wenigstens einen Zielsubstanz beaufschlagten magnetischen Beads ein Gelelektrophoreseprobenpuffer zugegeben wird,
dass das sich dabei bildende Gemisches auf 40 bis 95°C für 5 - 30 min erhitzt wird, dass die magnetischen Beads mittels eines Magneten aus dem Gemisch abgetrennt werden, und
dass der verbleibende Gelelektrophoreseprobenpuffer abpipettiert und in Geltaschen eines Elektrophoresegels aufgegeben und einer elektrophoretischen Auftrennung unterzogen wird, oder.
**dadurch gekennzeichnet, dass** die an die Beads gebundenen Zielsubstanzen durch Zugabe einer Carbonsäure eluiert werden,
wobei ein sich im Wege der Eluierung ergebendes Carbonsäureeluat vorzugsweise einrotiert und in einer Pufferlösung aufgenommen wird, oder
**dadurch gekennzeichnet, dass** die Zielsubstanz einem enzymatischen Verdau zugeführt werden, und dass die verdauten Zielsubstanzen einer massenspektrometrischen Identifizierung über Peptide Mass Fingerprint (PMF) oder MS/MS-Spektren unterzogen werden, oder
**dadurch gekennzeichnet, dass** die mit den Zielsubstanzen beaufschlagten magnetischen Beads gewaschen und anschließend einer Pufferlösung mit einer Protease zum Verdau der Zielsubstanzen zugeführt werden.

10. Verfahren nach Anspruch 9,
**dadurch gekennzeichnet, dass** eine massenspektrometrische Messung über Peptide Mass Fingerprint (PMF) oder MS/MS-Spektren der Pufferlösung nach dem enzymatischen Verdau durchgeführt wird, oder
**dadurch gekennzeichnet, dass** die Beads gewaschen werden anschließend einem Gemisch aus einem organischen Lösungsmittel und/oder Carbonsäuren zugegeben werden zum Erhalt eines Eluats,
wobei vorzugsweise eine massenspektrometrische Messung des Eluats durchgeführt wird.

11. Verfahren nach einem der Ansprüche 1 bis 10,
**dadurch gekennzeichnet, dass** die wenigstens eine an den Beads gebundene Zielsubstanz durch Zugabe einer Carbonsäure oder eines chaotropen Puffers eluiert wird und die sich in dem gewonnenen Eluat befindlichen Substanzen mit Hilfe von magnetischen, dendritischen, reversed-phase Partikel gebunden werden,
wobeidie mit den in dem Eluat befindlichen Substanzen beaufschlagten magnetischen, dendritischen, reversed-phase Partikel vorzugsweise einem Gelelektrophoreseprobenpuffer zugegeben werden,
dass das sich dabei bildende Gemisches auf 40 bis 95°C für 5 - 30 min erhitzt wird, dass die magnetischen Beads mittels eines Magneten aus dem Gemisch abgetrennt werden, und
dass der verbleibende Gelelektrophoreseprobenpuffer abpipettiert und in Geltaschen eines Elektrophoresegels aufgegeben und einer elektrophoretischen Auftrennung unterzogen wird, oder dass die mit den in dem Eluat befindlichen Substanzen beaufschlagten magnetischen, dendritischen, reversed-phase Partikel gewaschen und anschließend einer Pufferlösung mit einer Protease zum Verdau der an den Partikeln gebundenen Substanzen zugeführt werden.

## Claims

1. A method for purifying at least one target substance to be identified which is present or formed in a cell culture medium when cells are cultivated, in which magnetic particles, beads as they are known, which are functionalised on the surface thereof and to the surface of which the target substance selectively attaches, are added to the cell culture medium, and the particles to which the target substance is attached are selected from the cell culture medium by applying a magnetic field, **characterised by** the following method steps:
- providing a serum substitute which is obtained from a natural serum and is free or virtually free of low-molecular substances having a mass less than or equal to 60 kDa, in particular less than or equal to 10 kDa,
- adding the serum substitute to the cell culture medium which already contains the cells or to which the cells are added,
- incubating the cells in the cell culture medium enriched with serum substitute,
- separating at least some of the cell culture supernatant formed during the incubation process,
- filtering the cell culture supernatant by means of an ultrafiltration process so as to obtain a retentate,
- supplying the beads in such a way that the functionalised surface of the beads comprises a plurality of dendrimers containing up to 10 branches each, i.e. 10 generations, the terminal points of the last generation of each dendrimer being modified,
- adding the beads and a buffer solution to the retentate so as to form a mixture,
- incubating and binding the target substances contained in the retentate to the beads and magnetically selecting the magnetic beads out of the mixture.

2. The method according to claim 1, **characterised in that** the serum substitute is obtained as the retentate after ultrafiltration of a natural blood serum from an animal or human, and **in that** the retentate is washed with a growth medium or buffer, where Dulbecco's Modified Eagle Medium (DMEM), IMDM, IMEM, ERDF or RPMI1640 preferably is used as the growth medium wash solution.

3. The method according to any one of claims 1 or 2, **characterised in that** the serum substitute is obtained and provided in the following manner:
a) centrifuging natural serum using an ultrafiltration unit so as to obtain a supernatant, the retentate as it known, and a permeate,
b) adding a growth medium to the retentate and subsequently carrying out a centrifugation process according to step a),
c) repeating step b) n times,
d) adding growth medium to the retentate obtained so as to obtain a serum supernatant as it is known, and
e) filtering the serum supernatant so as to obtain a permeate corresponding to the serum substitute, wherein sterile filtration is preferably carried out when the serum supernatant is filtered, or wherein the reversible or irreversible protease inhibitor is added to the natural serum before step a).

4. The method according to claim 3, **characterised in that** phenylmethylsulphonyl fluoride (PMSF) and/or ethylenediamine tetraacetic acid (EDTA) and/or proteinogenic inhibitors are added to the serum individually as protease inhibitors or are added as a protease inhibitor cocktail.

5. The method according to claim 3, **characterised in that** the centrifugation process in steps a) and b) is carried out for at least 1 hour at ambient temperature down to a temperature of +4 °C, or
**characterised in that** step b) is repeated n times, n being 2 to 5, or
**characterised in that** the filtration process in step e) is carried out using a 0.2 µm sterile filter.

6. The method according to claim 1, **characterised in that** the cell culture supernatant is frozen at -20 °C to -80 °C before filtration, **in that** the frozen cell culture supernatant is thawed, and **in that** filtration is carried out by means of centrifugation using an ultrafiltration unit, or
**characterised in that** an antichaotropic buffer solution is used when adding the beads and the retentate, or
**characterised in that** after incubation and after the target substance contained in the retentate is bound to the beads and the beads have been magnetically selected, the beads provided with the target substance are washed with a low salt buffer solution, and **in that** the at least one target substance is subsequently eluted from the beads in a mixture containing at least one organic component and/or an acid, wherein alcohol or acetonitrile are used as the organic substance and carboxylic acid or mono-, di-, trihalogen carboxylic acid are used as the acid.

7. The method according to any one of claims 1 to 6, **characterised in that** Dulbecco's Modified Eagle Medium (DMEM), IMDM, IMEM, ERDF or RPMI1640 are used as the wash solution, and/or
**characterised in that** 1 to 10 % by volume of serum substitute is added to the cell culture medium when the serum substitute is added to the cell culture medium, and/or
**characterised in that** the following steps are carried out in order to supply the beads:
a) providing magnetic particles, beads for short, having a particle size on the nano- and/or micro-scale, i.e. 1 or more nm or µm, and comprising amino groups applied to the surfaces thereof,
b) reacting the beads in a methyl acrylate-containing organic solvent,
c) separating the beads from the organic solvent and washing the beads with an organic solvent,
d) reacting the beads in an ethylenediamine-containing organic liquid,
e) separating the beads from the organic liquid and washing the beads,
f) repeating steps b) to e) n times
g) suspending the beads in a solution containing at least one functionalising substance, and
h) separating and washing the beads and supplying the functionalised beads in water.

8. The method according to claim 7, **characterised in that** n is selected from 0 to 9, and/or **characterised in that** alcohol, R-OH for short, is used as the reaction liquid, R being C₁ to C₆ and/or **characterised in that** the suspension process is carried in an ultrasound bath and/or by heating to a maximum of 36 °C and/or by microwave synthesis, and/or
**characterised in that** halogen-containing solvents, to which activated alkyl derivatives (C₁ to C₃₀) are added, are used as the solution containing at least one functionalising substance, halogens, in particular Cl, Br, J, cyanates, isocyanates or isothiocyanates, being used as activated groups, or
**characterised in that**, after reaction step f), the beads are reacted with a bifunctional linker for amino groups, **in that** the beads are washed in a buffer, and **in that** proteins, e.g. antibodies at a concentration of c = 1 µg/ml to 50 mg/ml in a buffer solution are used as the at least one functionalising substance, wherein glutaric dialdehyde, bisulphosuccinimidyl suberate (BS3), disuccinimidyl suberate (DSS), dimethyl pimelimidate (DMP) or bifunctional PEG linkers are preferably used as bifunctional linkers, or
**in that** a phosphate or carbonate buffer is used as the washing buffer.

9. The method according to any one of claims 1 to 8, **characterised in that** the magnetic beads loaded with the at least one target substance are added to a gel electrophoresis sample buffer, **in that** the mixture thus formed is heated to 40 to 95 °C for 5 to 30 minutes, **in that** the magnetic beads are separated from the mixture by a magnet, and **in that** the remaining gel electrophoresis buffer is pipetted off and is added to gel slots of an electrophoresis gel, and undergoes electrophoretic separation, or
**characterised in that** the target substances bound to the beads can be eluted by adding a carboxylic acid, wherein the carboxylic acid eluate obtained from the elution process may be reduced in volume, and redissolved in an appropriate buffer solution, or
**characterised in that** the target substance undergoes enzymatic digestion, and **in that** the digested target substances undergo a mass spectrometric identification process by using peptide mass fingerprinting (PMF) or MS/MS spectra, or
**characterised in that** the magnetic beads loaded with the target substances are washed and subsequently added to a buffer solution containing a protease for digesting the target substances.

10. The method according to claim 9, **characterised in that** mass spectrometric measurements are carried out after the enzymatic digestion process by peptide mass fingerprinting (PMF) or MS/MS spectra, or
**characterised in that** the beads are washed and subsequently added to a mixture of an organic solvent and/or carboxylic acids so as to obtain an eluate, wherein mass spectrometric measurements preferably are carried out on the eluate.

11. The method according to any one of claims 1 to 10, **characterised in that** the at least one target substance bound to the beads is eluted by adding a carboxylic acid or a chaotropic buffer and the substances contained in the eluate obtained are bound using magnetic, dendritic, reversed-phase particles, wherein the magnetic, dendritic, reversed-phase particles loaded with the substances contained in the eluate are preferably added to a gel electrophoresis sample buffer, **in that** the mixture thus formed is heated to 40 to 95 °C for 5 to 30 minutes, **in that** the magnetic beads are separated from the mixture by a magnet, and **in that** the remaining gel electrophoresis sample buffer is pipetted off and added to gel slots in an electrophoresis gel and an electrophoretic separation process is carried out, or
that the magnetic, dendritic, reversed-phase particles loaded with the substances contained in the eluate are washed and subsequently added to a buffer solution containing a protease for digesting the substances bound to the particles.

## Revendications

1. Procédé de purification d'au moins une substance cible à mettre en évidence qui apparaît ou est formée dans un milieu de culture cellulaire lors d'une culture cellulaire, procédé selon lequel des particules magnétiques à surface fonctionnalisée appelées billes sont introduites dans le milieu de culture cellulaire, la substance cible se déposant de manière sélective sur leur surface et les particules dotées de la substance cible étant extraites du milieu de culture cellulaire par application d'un champ magnétique, **caractérisé par** les opérations suivantes :
- préparer un succédané de sérum obtenu à partir d'un sérum naturel et ne présentant pas ou peu de substances de faible poids moléculaire de masse inférieure ou égale à 60 kDa, notamment inférieure ou égale à 10 kDa ;
- mélanger le succédané de sérum au milieu de culture cellulaire qui contient déjà les cellules ou dans lequel les cellules ont déjà été introduites ;
- incuber les cellules dans le milieu de culture cellulaire enrichi par le succédané de sérum ;
- séparer au moins une partie d'un excédent de culture cellulaire formé lors de l'incubation ;
- filtrer l'excédent de culture cellulaire par ultrafiltration pour obtenir un rétentat ;
- préparer les billes de manière à ce que leur surface fonctionnalisée présente une pluralité de dendrimères ayant chacun jusqu'à dix ramifications à savoir dix générations, les parties terminales de chaque dernière génération de dendrimères étant modifiées ;
- mélanger les billes et une solution tampon au rétentat pour former un mélange ;
- incuber et lier les substances cibles contenues dans le rétentat aux billes et extraire les billes magnétiques du mélange par sélection magnétique.

2. Procédé selon la revendication 1,
**caractérisé par le fait que**
le succédané de sérum en tant que rétentat par ultrafiltration est obtenu à partir d'un sérum sanguin naturel animal ou humain, et que
le rétentat est lavé à l'aide d'un milieu nutritif ou d'un tampon, un milieu nutritif, Dulbecco's Modified Eagle Medium (DMEM), IMDM, IMEM, ERDF ou RPM11640 étant préférentiellement utilisé comme solution de lavage.

3. Procédé selon l'une des revendications 1 ou 2, **caractérisé par le fait que** le succédané de sérum est obtenu et préparé de la manière suivante :
a) centrifugeage de sérum naturel par l'intermédiaire d'une unité d'ultrafiltration pour l'obtention d'un surnageant, appelé le rétentat, et d'un perméat,
b) ajout du rétentat à un milieu nutritif et centrifugeage subséquent conformément à l'étape a)
c) n^{ème} répétition de l'étape b)
d) ajout d'un milieu nutritif au rétentat obtenu pour l'obtention de ce qu'on appelle le sérum surnageant et
e) filtration du sérum surnageant pour l'obtention d'un perméat correspondant au succédané de sérum, une filtration stérile étant préférentiellement exécutée lors de la filtration du sérum surnageant, ou le sérum naturel étant mélangé avant l'étape a) avec au moins un inhibiteur de protéase réversible ou irréversible.

4. Procédé selon la revendication 3, **caractérisé par le fait que** du fluorure de phénylméthylsulfonyle (PMSF) et/ou un acide diaminotétracarboxylique (EDTA) et/ou des inhibiteurs protéinogènes sont ajoutés comme inhibiteur de protéase au sérum individuellement ou comme cocktail d'inhibiteurs de protéase.

5. Procédé selon la revendication 3,
**caractérisé par le fait que** le centrifugeage est exécuté conformément aux étapes a) et b) pendant au moins une heure à température ambiante descendant jusqu'à +4°C, ou **caractérisé par le fait que** l'étape b) est répétée 2 à 5 fois à la suite, ou **caractérisé par le fait que** la filtration conformément à l'étape e) est exécutée avec un filtre stérile de 0,2 µm.

6. Procédé selon la revendication 1,
**caractérisé par le fait que** :
l'excédent de culture cellulaire est congelé dans le temps avant la filtration à une température située entre -20°C et -80° C,
l'excédent de culture cellulaire est décongelé,
la filtration par centrifugeage est exécutée en utilisant une unité d'ultrafiltration, ou
**caractérisé par le fait**
**qu'**une solution tampon antichaotropique est utilisée lors du mélange des billes ainsi que du rétentat, ou
**caractérisé par le fait que** :
après l'incubation, la liaison de la substance cible contenue dans le rétentat aux billes et la sélection magnétique des billes, les billes munies des substances cibles sont lavées avec une solution tampon à faible teneur en sel, et que au moins une substance cible est ensuite éluée par les billes dans un mélange constitué d'au moins un composant organique et/d'un acide, la substance organique utilisée étant préférentiellement de l'alcool ou de l'acétonitrile et l'acide étant préférentiellement de l'acide carboxylique ou de l'acide carboxylique mono-, di- ou trihalogéné.

7. Procédé selon une des revendications 1 à 6,
**caractérisé par le fait que** Dulbecco's Modified Eagle Medium (DMEM), IMDM, IMEM, ERDF ou RPM11640 sont utilisés comme milieu de culture cellulaire, et/ou **caractérisé par le fait que** lors du mélange du succédané au milieu de culture cellulaire, 1 à 10 % du volume de succédané de sérum est adjoint au milieu de culture cellulaire, et/ou
**caractérisé par le fait que** les étapes suivantes ont été exécutées pour préparer les billes :
a) préparation de particules magnétiques, appelées en abrégé des billes, avec une taille de particule de l'ordre du nanomètre et/ou du micromètre, c'est-à-dire un ou plusieurs nm ou µm, et avec des groupes amino appliqués sur leurs surfaces,
b) réaction des billes dans un solvant organique contenant de l'acrylate de méthyle,
c) séparation des billes d'un solvant organique et lavage des billes avec un solvant organique,
d) réaction des billes dans un liquide organique contenant de l'éthylène diamine,
e) séparation des billes du liquide organique et lavage des billes,
f) n^{ème} répétition des étapes b) à e)
g) suspension des billes dans une solution contenant au moins une substance fonctionnelle, et
h) séparation et lavage des billes et préparation des billes fonctionnalisées dans l'eau.

8. Procédé selon la revendication 7,
**caractérisé par le fait que** n est choisi entre 0 et 9, et/ou **caractérisé par le fait que** de l'alcool, en abrégé R-OH, est utilisé comme liquide de réaction, avec R = C₁ à C₆, et/ou
**caractérisé par le fait que** la suspension est exécutée dans le cadre d'un bain à ultrasons et/ou par chauffage à 36°C maximum et/ou dans le cadre d'une synthèse par micro-ondes, et/ou
**caractérisé par le fait que** des solvants halogénés sont utilisés, moyennant l'ajout de dérivés alkylés activés (C₁ - C₃₀), comme substance contenant au moins une substance fonctionnelle, des halogènes, en particulier Cl, Br, J, cyanat, isocyanate ou isothiocyanate, étant utilisés comme groupes activés, ou
**caractérisé par le fait que**, après l'étape de procédé f), les billes sont amenées à réagir avec un lieur bi-fonctionnel pour groupes amino, que les billes sont lavées dans un tampon, et qu'un anticorps d'une concentration de c = 1 mg/ml à 50 mg/ml est utilisé comme la solution comprenant au moins une substance fonctionnelle dans une solution tampon, préférentiellement du glutardialdéhyde, du subérate de bissulfosuccinimidyle (BS3), du disuccinimidyl subérate (DSS), du diméthyle pimélimidate (DMP) ou un lieur bi-fonctionnel PEG étant utilisé comme lieur bi-fonctionnel, ou un tampon de phosphate ou de carbonate étant préférentiellement utilisé comme tampon de lavage.

9. Procédé selon une des revendications 1 à 8,
**caractérisé par le fait :**
**qu'**un tampon échantillon d'électrophorèse sur gel est ajouté aux billes magnétiques alimentées avec au moins une substance cible,
**que** le mélange se formant alors est réchauffé à une température située entre 40 et 95°C pendant 5 à 30 min, que les billes magnétiques sont séparées du mélange à l'aide d'un aimant, et
**que** le tampon échantillon d'électrophorèse sur gel restant est retiré à la pipette et est déposé dans des poches de gel d'un gel d'électrophorèse et est soumis à une séparation par électrophorèse, ou
**caractérisé par le fait que** les substances cibles liées aux billes sont éluées par addition d'un acide carboxylique, un éluat d'acide carboxylique résultant de l'éluation étant préférentiellement extrait par évaporation rotationnelle et inséré dans une solution tampon, ou
**caractérisé par le fait que** la substance cible est amenée à une digestion enzymatique, et que les substances digérées sont soumises à une identification spectrométrique de masse par empreinte peptidique massique [Peptide Mass Fingerprint (PMF)] ou à des spectres MS/M, ou
**caractérisé par le fait que** les billes magnétiques alimentées avec les substances cibles sont lavées puis amenées à une solution tampon avec une protéase assurant la digestion des substances cibles.

10. Procédé selon la revendication 9,
**caractérisé par le fait**
**qu'**une mesure spectrométrique de masse par empreinte peptidique massique [Peptide Mass Fingerprint (PMF)] ou des spectres MS/MS de la solution tampon sont exécutés après la digestion enzymatique, ou
**caractérisé par le fait que** les billes sont lavées, puis ajoutées à un mélange de solvant organique et/ou d'acides carboniques afin d'obtenir un éluat, une mesure spectrométrique de masse de l'éluat étant préférentiellement exécutée.

11. Procédé selon une des revendications 1 à 10,
**caractérisé par le fait**
**qu'**au moins une substance cible liée aux billes est éluée par ajout d'un acide carboxylique ou d'un tampon chaotropique et
**que** les substances se trouvant dans l'éluat obtenu sont liées à l'aide de particules magnétiques, dendritiques, à polarité de phases inversées, les particules magnétiques alimentées avec les substances se trouvant dans l'éluat, dendritiques, à polarité de phases inversées sont préférentiellement ajoutées à un tampon échantillon d'électrophorèse sur gel,
**que** le mélange se formant alors est réchauffé à une température située entre 40 et 95°C pendant 5 à 30 min,
**que** les billes magnétiques sont séparées du mélange à l'aide d'un aimant, et
**que** le tampon échantillon d'électrophorèse sur gel restant est retiré à la pipette et est déposé dans des poches de gel d'un gel d'électrophorèse et est soumis à une séparation par électrophorèse, ou
**que** les particules magnétiques alimentées avec les substances se trouvant dans l'éluat, magnétiques, dendritiques, à polarité de phases inversées sont lavées puis amenées à une solution tampon avec une protéase assurant la digestion des substances cibles.
